# EUROPEAN PATENT SPECIFICATION

(11) **EP 4 601 718 B1**
(45) Date of publication and mention of the grant of the patent: **25.11.2026**
(21) Application number: 23789642.8
(22) Date of filing: 12.10.2023
(51) Int. Cl.: A61M 5/142

(54) **FILLING APPARATUS AND METHOD FOR FILLING A REUSABLE RESERVOIR OF A MEDICAL DISPENSING DEVICE**
FÜLLVORRICHTUNG UND VERFAHREN ZUM BEFÜLLEN EINES WIEDERVERWENDBAREN VORRATSBEHÄLTERS EINER MEDIZINISCHEN ABGABEVORRICHTUNG
APPAREIL DE REMPLISSAGE ET PROCÉDÉ DE REMPLISSAGE D'UN RÉSERVOIR RÉUTILISABLE D'UN DISPOSITIF DE DISTRIBUTION MÉDICAL

(30) Priority: 14.10.2022 EP 22201703
(43) Date of publication of application: 20.08.2025
(73) Proprietor: F. Hoffmann-La Roche AG, 4070 Basel (CH); Roche Diabetes Care GmbH, 68305 Mannheim (DE)
(72) Inventor: BAUER, Andreas, 68305 Mannheim (DE); NORRICK, Nicklas, 68305 Mannheim (DE); MARKL, Kristina, 68305 Mannheim (DE)
(74) Representative: Pfaffmann, Lucas
(86) International application number: PCT/EP2023/078314
(87) International publication number: WO 2024/079250

(56) References cited:
- US-A1- 2014 010 727
- US-A1- 2015 151 041
- US-B2- 8 839 828

## Description

The present invention relates to a filling apparatus for filling a reusable reservoir of a medical dispensing device, the filling apparatus comprises a receiving portion that is configured for holding a reusable reservoir of a medical dispensing device, a supply container that is configured for containing a liquid medication, a first connection port that is configured for fluidly connecting an inner volume of the reusable reservoir of a medical dispensing device with the supply container, and a sterilization device that is configured for sterilizing the inner volume of the reusable reservoir of a medical dispensing device.

A hitherto known medical dispensing device comprises a reservoir that is configured for containing a liquid medication. During use of the medical dispensing device, the liquid medication is dispensed out of the reservoir through an opening therein and administered to a patient. However, after being depleted, the empty reservoir is disposed thereby increasing an amount of waste stressing the environment.

When providing a reusable reservoir instead of a disposable reservoir less waste is created. However, care has to be taken that refilled reservoir can unproblematically be used and for example not contaminated which would may harm a user of the medical dispensing device.

US 8,839,828 B2 discloses a system for filling a reusable medical reservoir of a medication dosing apparatus. Said filling device further comprises a purification device allowing a sterile cleansing of the reusable medication reservoir before refilling it with medication. For sterile cleansing a cleansing agent, such as an isotonic table salt solution or an alcoholic solution, is provided in a container or supply tank of the filling apparatus. This design has the disadvantage that a residual amount of medication and/or a residual amount of the cleansing agent may remain in the reusable medical reservoir after being cleaned. When refilling the medical reservoir the residual amount of cleansing agent and/or residual amount of liquid medication is mixed with the newly filled medication. This lowers the quality of the refilled reusable medical reservoir, and makes refilling of the reusable medical reservoir less attractive to a user.

Accordingly, it is an object of the present invention to provide a filling device that avoids at least one of the problems associated with filling apparatuses known from the prior art. In particular, it is an object of the present invention to provide an improved filling apparatus for sterilizing a reusable reservoir of a medical dispensing device.

At least one of the above objects is solved by the provision of the subject matter disclosed in the claims and throughout the specification.

According to a first aspect, a filling apparatus for filling a reusable reservoir of a medical dispensing device is provided. The filling apparatus comprises a receiving portion that is configured for holding a reservoir of a medical dispensing device, a supply container that is configured for containing a liquid medication, a first connection port that is configured for fluidly connecting an inner volume of the reusable reservoir of a medical dispensing device with the supply container, and a sterilization device that is configured for sterilizing the inner volume of the reusable reservoir of a medical dispensing device. The filling apparatus further comprises a discharging device that is configured to discharge deposit, during use of the filling apparatus, from of the reusable reservoir of the medical dispensing device. The discharging device is configured for fluidly connecting the inner volume of the reusable reservoir of a medical dispensing device with the discharging device. The sterilization device comprises a radiation chamber that is configured for housing the reusable reservoir of a medical dispensing device. The radiation chamber comprises an access opening that is configured to be opened and closed, and a protective barrier that is configured to hinder radiation from leaving the radiation chamber. The sterilization device further comprises a radiation source that is configured for providing radiation within the radiation chamber.

During use, a reusable reservoir is inserted into the radiation chamber of the filling apparatus via the access opening of the radiation chamber. The access opening of the radiation chamber is configured so that it can be opened and closed for placing the reusable reservoir in the radiation chamber and for removing the reusable reservoir from the radiation chamber. The sterilization device can further comprise a safety-mechanism, for example a safety-switch, that hinders radiation spreading within the radiation chamber when the access opening of the radiation chamber is open. The safety-mechanism can switch-off and/or hinder activation of the radiation source in case the access opening of the radiation chamber is open so not to generate radiation and/or so as not to provide radiation to the radiation chamber. The safety-mechanism increases the safety of organisms and the environment near the filling apparatus by preventing at least an excessive exposure of the organisms and the environment to radiation leaving the radiation chamber.

When being placed in the radiation chamber, the reusable reservoir is brought into engagement with the receiving portion. The receiving portion holds the reusable reservoir in a predetermined positon within the radiation chamber. The connecting port is coupled to an opening of the reusable reservoir thereby fluidly connecting the inner volume of the reusable reservoir to a supply container. The supply container is configured for storing a liquid medication so that the reusable reservoir can filled or refilled, respectively, with liquid medication from the supply container. Liquid medication in the context of the present invention refers to a liquid drug or pharmaceutically effective solution. For instance, the liquid medication can be insulin, or a solution containing insulin.

By way of example only, the supply container can be a replaceable supply container and/or a refillable supply container that is fixedly integrated into the filling apparatus. The replaceable container can be disposable or reusable. A replaceable, disposable supply container can comprise a tamper-evident closure indicating a first-time opening or first-time use of the disposable supply container. Alternatively or in addition, the tamper-evident closure of the disposable supply container can hinder a refilling of the disposable supply container. These designs of a supply container help to provide a larger amount of liquid medication for filling and refilling a reusable reservoir of a medical dispensing device in a safe and convenient way. A tamper-evident closure further improves the patient's safety by hindering use of a fraudulent/manipulated user reservoir.

After use of the reusable reservoir and its depletion by a medical dispensing device, deposit can still be present in the reusable reservoir. Thus, before refilling the reusable reservoir deposit that may be contained in the reusable reservoir is removed from the reusable reservoir by the discharging device of the filling apparatus. In the context of the present invention, deposit refers to one or more of a residual amount of liquid medication, a residual amount of a cleansing agent, a residual amount of bodily fluids, and/or a residual amount of contaminations in the reusable reservoir as well as combinations thereof.

The radiation chamber is part of the sterilization device included in the filling apparatus and comprises a protective barrier that is configured to hinder radiation from leaving the radiation chamber. The protective barrier helps to protect organisms near the filling apparatus, in particular a user of the filling apparatus, against radiation that, during use of the sterilization device is generated by the radiation source for sterilizing the reusable reservoir. The protective barrier limits the presence of radiation outside the filling apparatus that is generated by the radiation source and that may otherwise harm user of the filling apparatus or the environment close to the filling apparatus. The selection of the radiation source is tuned to the selection of the protective barrier, and vice versa. For instance, the radiation source can comprise or form a source for UV-C radiation and/or a source for gamma radiation and/or source for X-ray radiation. Accordingly, the protective barrier can be configured to hinder the respective UV-C radiation and/or gamma radiation and/or X-ray radiation that, during use of the filling apparatus is provided by the radiation source passing the protective barrier of the radiation chamber.

In the meaning of the present invention, UV-C radiation refers to an electromagnetic wave having a wavelength in the range between equal to or more than 100 nm and equal to or less than 280 nm. In a variant, the radiation source is configured to generate UV-C radiation in the range between equal to or more than 265 nm and equal to or less than 270 nm. According to an embodiment, the radiation source is configured to provide a median radiation efficiency of UV-C radiation within the radiation chamber in the range between equal to or more than 10 J/m² and equal to or less than 450 J/m². Alternatively, the median radiation source is configured to provide a median radiation efficiency within the radiation chamber in the range between equal to or more than 10 J/m² and equal to or less than 50 J/m².

A source for gamma radiation, in the meaning of the present invention, is a radiation source that is configured for providing an electromagnetic wave having a wavelength equal to or less than 0.005 nm and more than 0 nm.

A source for X-ray radiation, in the meaning of the present invention, is a radiation source that is configured for providing an electromagnetic wave having a wavelength in the range between equal to or more than 0.1 nm and equal to or less than 10 nm.

According to an embodiment, the respective radiation source is configured to provide a median radiation dose within the radiation chamber which is necessary to achieve a sterility assurance level of 10⁻⁶, meaning that after the sterilizing of the reservoir within the radiation chamber only one or less microorganism out of one million microorganisms of the germ *Bacillus pumilus* remains active. The median radiation dose within the radiation chamber describes the absorbed radiation dose necessary to achieve the aforementioned sterility assurance level of 10⁻⁶. For example, a respective radiation source is configured to provide a median radiation dose of UV-C radiation and/or of gamma radiation and/or X-ray radiation within in the radiation chamber in the range between equal to or more than 25 kJ/kg and equal to or less than 32 kJ/kg.

According to an embodiment, the protective barrier is integrated into housing elements forming the radiation chamber. Alternatively or in addition, the protective barrier forms the housing elements of the radiation chamber or form at least a circumferential layer of the housing elements of the radiation chamber. In the meaning of the present invention, housing elements inter alia refer to wall elements and door elements defining a closed dosing chamber. "Closed" in the context of the present invention does not exclude presence of supply lines, connectors and tubing for operating the radiation chamber as a component of the filling apparatus. Hindering radiation from leaving the closed radiation chamber is achieved, when the respective median radiation dose of the radiation as generated by the respective radiation source is outside of the radiation chamber at a distance of 1 cm from the closed access opening of the radiation chamber less than 5 %, preferably less than 1 %, of the median radiation dose inside of the radiation chamber.

The radiation source can be housed within the radiation chamber or be placed outside the radiation chamber. When being placed outside the radiation chamber, the radiation source can comprise an additional protective barrier hindering radiation from leaving the filling apparatus. In any event, the sterilizing device is configured such, that during use of the filling apparatus, radiation originating from the radiation source is spread within the radiation chamber for sterilizing the reusable reservoir.

A medical dispensing device is a device for dispensing a medication, preferably liquid medication, to a patient. By way of example, the medical dispensing device can be a medical infusion pump for infusing liquid medication into a patient's body. A medical infusion pump, for example, can be a body-wearable infusion pump. Accordingly, a reusable reservoir of a medical dispensing device can be a reusable reservoir of a medical infusion pump. In an embodiment, the medical dispensing device is an insulin patch pump, and the reusable reservoir is a reusable reservoir of the insulin patch pump.

In an embodiment, the discharging device comprises a waste container that is configured for storing deposit that during use of the discharging device is discharged from the reusable reservoir of a medical dispensing device. For example, the waste container is a removable and disposable container. Deposit discharged from the reusable reservoir is stored in the waste container, and, thus, can be disposed together with the waste container in a clean and sterile manner.

The filling apparatus can further comprise a drive mechanism that is configured for actuating a discharging mechanism of the reusable reservoir. For instance, a discharging mechanism of a reusable reservoir can comprise a plunger that is movably arranged within the inner volume of the reusable reservoir, wherein upon moving the plunger within the reusable reservoir the inner volume can be increased or decreased. Upon decreasing the inner volume of the reusable reservoir, deposit or content within the reusable reservoir is discharged from the reusable reservoir for instance into the waste container of the discharging device. Likewise, when increasing the inner volume of the reusable reservoir liquid medication can be conveyed from the supply container via the connection port into the reusable reservoir.

In an embodiment, the discharging device comprises a heating module that is configured for evaporating deposit in the inner volume of the reusable reservoir of the medical dispending device. For example, the heating module can comprise a heating coil and/or a heat lamp. Once deposit is evaporated by the heating module, during use of the filling apparatus, the evaporated, and thus, gaseous deposit can be easily discharged from the reusable reservoir.

According to an embodiment, the first connection port is configured for alternately fluidly connecting the inner volume of the reusable reservoir with the supply container and fluidly connecting the inner volume of the reusable reservoir with a waste container of the discharging device. The connection port can comprise a valve that is configured to be operated in a discharging state and a supply state. In the discharging state, the valve of the connection port fluidly connects the inner volume of the reusable reservoir to the waste container. In the supply state, the valve of the connection port fluidly connects the inner volume of the reusable reservoir to the supply container. A connection port comprising a valve that is configured for alternately fluidly connecting the inner volume of the reservoir to either a waste container or a supply container helps to provide a compact and simple to use filling apparatus.

The filling apparatus can further comprise a cleansing agent container that is configured for containing a cleansing agent for cleansing a reusable reservoir of a medical dispensing device. During use of the filling apparatus, the cleansing agent container is fluidly connected to the inner volume of the reservoir. For example, a cleansing agent is one or more of isopropyl alcohol, ethanol, distilled water, or ethylene oxide gas.

If respectively present, the supply container and/or the waste container and/or the cleansing agent container can be fluidly connected to the first connection port, so that, during use of the filling apparatus, the respective containers are fluidly connected to the inner volume of the reusable reservoir by the first connection port. For alternately fluidly connecting the respective container to the inner volume of the reusable reservoir, the first connection port can comprise a valve that is configured for alternately fluidly coupling one at time of the respective containers to the inner volume of the reusable reservoir. Alternatively, the filling apparatus can comprise a second connection port that is configured for fluidly connecting the waste container to the inner volume of the reusable reservoir and/or a third connection port that is configured for fluidly connecting the cleansing agent container to the inner volume of the reusable reservoir. Likewise, the reusable reservoir can comprise a first opening that is configured for coupling the inner volume of the reusable reservoir to the first connection port. In addition, the reusable reservoir can comprise a second opening that is configured for coupling the inner volume of the reusable reservoir to the second connection port. The second connection port can be configured to fluidly couple the inner volume of the reusable reservoir via the second opening of the reusable reservoir to a waste container and/or the cleansing agent container. Further in addition, the reusable reservoir can comprise a third opening that is configured for coupling the inner volume of the reusable reservoir to the third connection port. The third connection port can be configured to fluidly couple the inner volume of the reusable reservoir via the third opening of the reusable reservoir to a cleansing agent container.

In an embodiment, the cleansing device and the discharging device are fluidly connected to the inner volume of the reusable reservoir by two different connection ports. For example, the cleansing agent container can be fluidly connected to the inner volume of the reusable reservoir via the first connection port, while the waste container of the discharging device is fluidly connected to the inner volume of the reusable reservoir via the second connection port. During use of the filling apparatus, the inner volume of the reusable reservoir is flushed with a cleansing agent provided by the cleansing agent container through one of the two connection ports, while cleansing agent is removed from the inner volume of the reusable reservoir via the other of the two connection ports. Thereby, a direction of flow of the cleansing agent is achieved within the inner volume of the reusable reservoir from one connection port to the other connection port. For example, during use, cleansing agent from the cleansing agent container is flowing through the first connection port into the inner volume of the reusable reservoir and from the inner volume of the reusable reservoir via the second connection port into the waste container. A flow of cleansing agent within the inner volume of the reusable reservoir from one connection port to another connection port allows a continuous flow of cleansing agent through the reservoir that can sweep along gas bubbles and deposit out of the inner volume of the reservoir. In an embodiment the two connection ports are arranged that, in a state in which the filling apparatus is placed on a table ready-for-use, the connection port fluidly connected to the cleansing agent container is closer to the geocenter than the connection port fluidly connected to the waste container. Thereby, the flow of cleansing agent within the inner volume is directed at least in part against the gravity, such that gas bubbles that may be contained in the inner volume of the reusable reservoir during cleansing of the inner volume are already naturally tending to rise in the direction of the connection port fluidly connected to the waste container.

According to an embodiment, the filling apparatus further comprises a charging device for charging a rechargeable battery of a reusable reservoir and/or for charging a rechargeable battery of a medical dispensing device. The filling apparatus can comprise a docking station that is configured for electrically connecting the charging device to a rechargeable battery of a medical dispensing device.

The present invention further relates to medical dispensing device system comprising a reusable reservoir for a medical dispensing device, and a filling apparatus for filling the reusable reservoir of a medical dispensing device, the filling apparatus comprises:
- a receiving portion that is configured for holding a reservoir of a medical dispensing device;
- a supply container that is configured for containing a liquid medication;
- a first connection port that is configured for fluidly connecting an inner volume of the reservoir of a medical dispensing device with the supply container;
- a sterilization device that is configured for sterilizing the inner volume of the reservoir of a medical dispensing device, wherein
- the filling apparatus further comprises a discharging device that is configured to discharge deposit, during use of the filling apparatus, from the reusable reservoir of the medical dispensing device
   ∘ the discharging device is configured for fluidly connecting the inner volume of the reusable reservoir of a medical dispensing device with the discharging device;
- and further in that the sterilization device comprises a radiation chamber that is configured for housing the reusable reservoir of a medical dispensing device; the radiation chamber comprises
   ∘ an access opening that is configured to be opened or closed; and
   ∘ a protective barrier that is configured to hinder radiation from leaving the radiation chamber;
- and in that the sterilization device comprises a radiation source that is configured to providing radiation within the radiation chamber.

In an embodiment, the reusable reservoir comprises an opening that is configured for fluidly connecting the inner volume of the reusable reservoir to the first connection port. The reusable reservoir can further comprise a removable plug portion comprising a septum, when being inserted into the opening the plug portion sealing the opening fluidly tight, but still allowing access to the inner volume of the reusable reservoir through the septum, for instance by a connecting needle of the medical dispensing device. When being removed the at least one opening allows access to the inner volume of the reservoir via the first connection port.

The reusable reservoir can be a reusable reservoir of a body-wearable insulin infusion pump, such as for example an insulin patch pump, wherein the medical dispensing device system comprises a medical dispensing device that is a body-wearable insulin infusion pump. In an embodiment, the radiation chamber is configured to house the body-wearable insulin infusion pump alone or in a state separate to the reusable reservoir or in a state including the reusable reservoir.

According to an embodiment, the radiation source of the filling apparatus of the medical dispensing device system comprises or is one or more of a source for UV-C radiation, a source for gamma radiation or a source for X-ray radiation, wherein the reusable reservoir is configured to be transparent for the respective radiation provided by the radiation source. Transparent for the respective radiation provided by the radiation source in the meaning of the present invention means that when the radiation provided by the radiation source encounters the reusable reservoir almost all, preferably at least 70%, of the radiation passes through the reusable reservoir into the inner volume of the reusable reservoir. In an embodiment, the reusable reservoir is at least partially made from glass and/or a transparent plastic material that is transparent for electromagnetic waves having a wavelength in the range from equal to or larger than 400 nm and equal to or less than 780 nm. This designs not only allows visible inspection of the inner volume of the reservoir by a human eye, but also allows radiation generated by the respective radiation source to reach the inner volume of the reservoir by passing the material of the reusable reservoir.

Insofar, as in the foregoing as well as in the following detailed description of embodiments and claims of a filling apparatus for filling a reusable reservoir reference is made to a filling apparatus or to a reusable reservoir of a medical dispensing device, the characteristics described thereof are also applicable for the filling apparatus as part of a medical dispensing device system or for the reusable reservoir as part of a medical dispensing device system, respectively.

The present invention further relates to a method for filling a reusable reservoir of a medical dispensing device, the method is constituted to be executed by a filling apparatus of the present invention, the method comprises the steps of:
∘ discharging deposit from a reusable reservoir by the discharging device of the filling apparatus;
∘ sterilizing the inner volume of the reusable reservoir by providing radiation within the radiation chamber by the radiation source of the sterilization device of the filling apparatus; and
∘ filling the inner volume of the reusable reservoir by providing liquid medication from the supply container of the filling apparatus via the first connection port of the filling apparatus.

First, deposit that may still be contained in the inner volume of the reusable reservoir is removed by the discharging device of the filling apparatus. The deposit is discharged from the inner volume of the reusable reservoir into the waste container so that is can be disposed in a clean and simple way. The then emptied reusable reservoir is sterilized in the by sterilizing the inner volume of the reusable reservoir by providing radiation within the radiation chamber by the radiation source of the sterilization device of the filling apparatus. Afterwards, the inner volume of the reusable reservoir is filled with liquid medication from the supply container of the filling apparatus via the first connection port of the filling apparatus.

The method for filling a reusable reservoir of a medical dispensing device can further comprise the step of evaporating residual deposit in the inner volume of the reusable reservoir by a heating module of the filling apparatus.

Further optional, the method for filling a reusable reservoir of a medical dispensing device can comprise the step of cleansing the inner volume of the reusable reservoir by flushing the inner volume of the reusable reservoir with a cleansing agent provided by a cleansing agent container of the filling apparatus. Flushing the inner volume of the reusable reservoir helps to completely residual deposit from the inner volume of the reusable reservoir, and, thus, helps to provide a safely reusable reservoir.

In an embodiment, the method for filling a reusable reservoir of a medical dispensing device comprises the step of charging a rechargeable battery of the reusable reservoir and/or charging a rechargeable battery of a medical dispensing device by a charging device of the filling apparatus.

Insofar, as in the foregoing as well as in the following detailed description of embodiments and claims of a method for filling a reusable reservoir of a medical dispensing device by a filling apparatus of the present invention reference is made to a filling apparatus or to a reusable reservoir of a medical dispensing device, the characteristics described thereof are respectively applicable for the filling apparatus alone and as part of a medical dispensing device, or the reusable reservoir alone and as part of a medical dispensing device..

Further advantages, features and technical effects of the present invention are apparent from the following description of embodiments and the accompanying figures.
- Figure 1: shows a schematic view of a medical dispensing device system according to an embodiment of the present invention;
- Figure 2: shows a schematic view of a medical dispensing device system according to another embodiment of the present invention; and
- Figure 3: shows a schematic view of a medical dispensing device system according to another embodiment of the present invention.

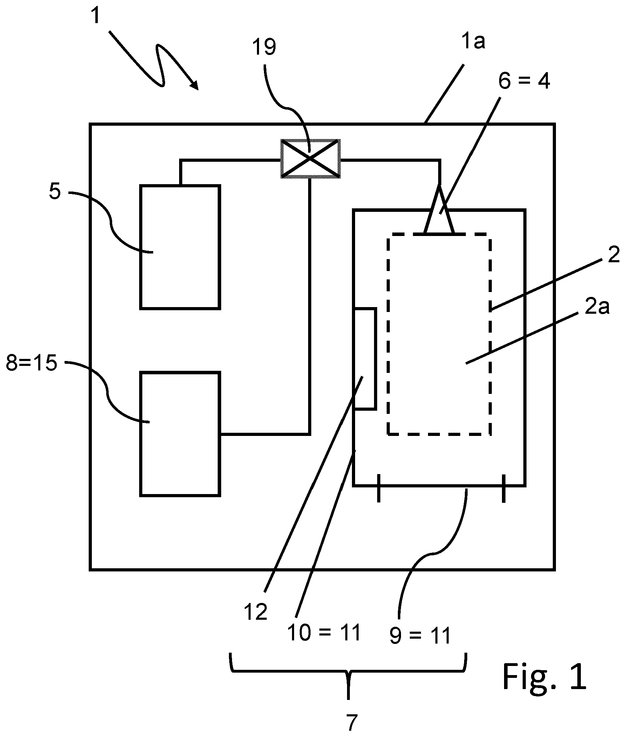

Figure 1 shows a schematic view of a medical dispensing device system 1 according to an embodiment of the present invention. The medical dispensing device system 1 comprises a reusable reservoir 2 for a medical dispensing device and a filling apparatus 1a that is configured for discharging deposit that may be contained in an inner volume 2a of the reusable reservoir 2, sterilizing the emptied inner volume 2a of the reusable reservoir 2 and filling the inner volume 2a of the reusable reservoir 2 with liquid medication so that the reusable reservoir 2 can be reused in a medical dispensing device 3 (not shown).

The medical dispensing device system 1 further comprises a pump base (not shown) that together with the reusable reservoir 2 form a medical dispensing device 3 (not shown).

The filling apparatus 1a comprises a discharging device 8 that is configured to discharge deposit, during use of the filling apparatus 1a, from the inner volume 2a of the reusable reservoir 2. The discharging device 8 comprises a waste container 15 that is configured for storing deposit discharged from the inner volume 2a of the reusable reservoir 2. The waste container 15 is disposable, removable container that can be disposed together with the deposit discharged from the inner volume 2a of the reusable reservoir 2. The discharging device 8 is fluidly connected to the inner volume 2a of the reusable reservoir 2 via a first connection port 6. The first connection port 6 provides access to the inner volume 2a of the reusable reservoir 2 via an opening of the reusable reservoir 2.

The filling apparatus 1a further comprises a sterilization device 7 that is configured for sterilizing the inner volume 2a of the reusable reservoir 2. The sterilization device 7 comprises a radiation chamber 9 housing the reusable reservoir 2 when being received in a receiving portion 4 of the filling apparatus 1a. The receiving portion 4 holds the reusable reservoir 2 in place within the radiation chamber 9 during discharging, sterilizing and filling the inner volume 2a of the reusable reservoir 2. For sterilizing the inner volume 2a of the reusable reservoir 2, the sterilization device 7 further comprises a radiation source 12 that is configured to provide radiation within the radiation chamber 9 during use of the filling apparatus. The radiation provided by the radiation source 12 is selected so to achieve a sterility assurance level of 10⁻⁶. However, for protecting organisms close to the filling apparatus 1a against radiation provided by the radiation source 12, the radiation chamber 9 comprises a protective barrier 11. The protective barrier 11 hinders radiation from leaving the radiation chamber 9. The radiation chamber 9 comprises an access opening 10 that is configured to be opened and closed. When opened, the access opening 10 allows a reusable reservoir 2 to be inserted into the radiation chamber 9 and to be removed from the radiation chamber 9. When the access opening 10 is closed, the closed access opening 10 together with the protective barrier 11 hinders radiation leaving the radiation chamber 9.

After being sterilized, the inner volume 2a of the reusable reservoir 2 is filled with liquid medication. The filling apparatus 1a comprises a replaceable supply container 5 that is configured for containing the liquid medication. The supply container 5 is fluidly connected to the inner volume 2a of the reusable reservoir 2 via the first connection port 6. In order to either discharge deposit from the inner volume 2a from the reusable reservoir 2 or to fill liquid medication into the inner volume 2a from the reusable reservoir, the first connection port 6 is fluidly coupled to a valve 19. The valve 19 is configured to fluidly connect first connection port 6 alternately to either the supply container 5 or to the waste container 15.

Accordingly, the filling apparatus (1a) is configured for carry out a method for filling a reusable reservoir (2) of a medical dispensing device (3) comprising the steps of discharging deposit from a reusable reservoir (2) by the discharging device (8) of the filling apparatus; sterilizing the inner volume (2a) of the reusable reservoir (2) by providing radiation within the radiation chamber (9) by a radiation source (12) of a sterilization device (7) of the filling apparatus (1a); and filling the inner volume (2a) of the reusable reservoir (2) by providing liquid medication from the supply container (5) of the filling apparatus (1a) via the first connection port (6) of the filling apparatus (1a). Thus, the method is constituted to be executed by the filling apparatus (1a).

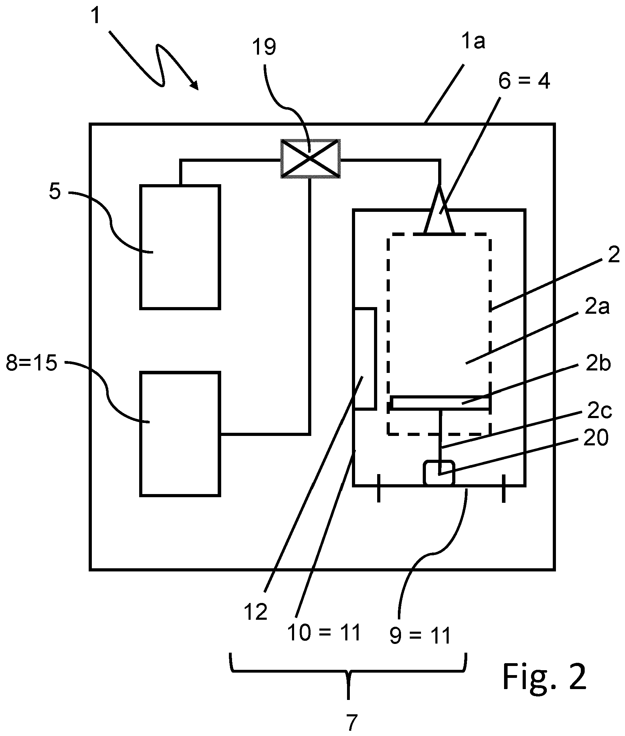

Figure 2 shows a schematic view of a medical dispensing device system 1 according to another embodiment of the present invention. This embodiment of the medical device system 1 differs from the embodiment of the medical device dispensing device system 1 according to figure 1 in the filling apparatus 1a additionally comprising a drive mechanism 20. Furthermore, the reusable reservoir 2 further comprises a plunger 2b and a plunger rod 2c.

During use of the filling apparatus 1a, the plunger rod 2c is operatively coupled to the drive mechanism 20. The drive mechanism 20 is configured to actuate the plunger rod 2c which again is configured to move the plunger 2b within the inner volume 2a of the reusable reservoir 2. By moving the plunger 2b within the inner volume 2a of the reusable reservoir 2, the inner volume 2a is increased or decreased. By decreasing the inner volume 2a, deposit which is may present in the inner volume 2a will be discharged via the first connection port 6 into the waste container 15. Likewise, when moving the plunger 2b within the inner volume 2a of the reusable reservoir 2 so to increase the inner volume 2a liquid medication from the supply container 5 can be conveyed into the inner volume 2a of the reusable reservoir 2 via the first connection port 6.

The first connection port 6 and/or valve 19 according to this embodiment, further comprises an air-vent, the air vent is configured to fluidly connect the inner volume 2a of the reusable reservoir 2 to ambient air, thereby allowing to move the plunger 2b within the inner volume 2a without discharging deposit to the waste container 15 and without filling the inner volume 2a with liquid medication from the supply container 5. This design allows to provide an empty inner volume 2a of its maximum volume so that the radiation can pass through the inner volume 2a of the reservoir 2 along a direction perpendicular to the direction of movement of the plunger 2b without being substantially hindered by the plunger 2b and/or the plunger rod 2c.

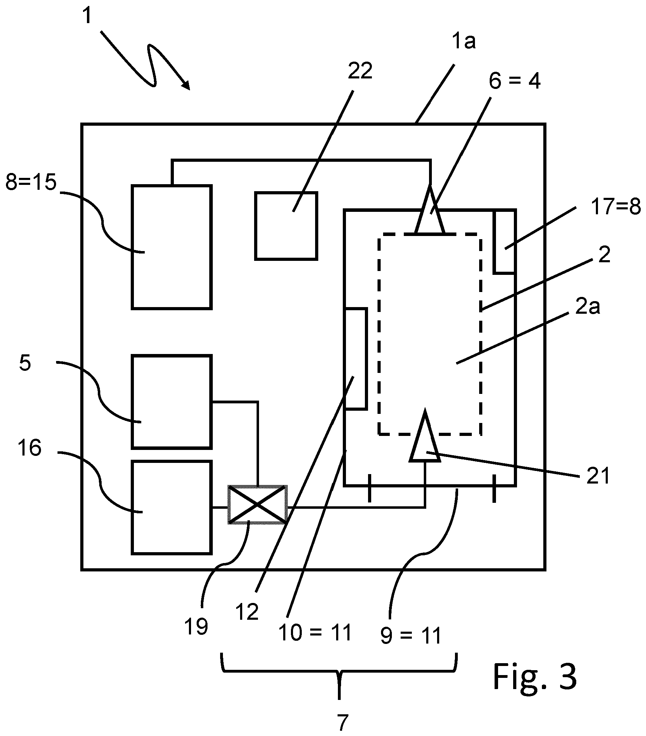

Figure 3 shows a schematic view of a medical dispensing device system 1 according to yet another embodiment of the present invention. The embodiment of the medical dispensing device system 1 as shown in figure 3 differs from the embodiment of the medical dispensing device system 1 as shown in figure 1, in comprising a first connection port 6 that is configured to fluidly connect the inner volume 2a of the reusable reservoir 2 to the waste container 15. The supply container 5 is fluidly connected to the inner volume 2a of the reusable reservoir 2 via a second connection port 21. Second connection port 21 is, thus, configured to fluidly connect the inner volume 2a of the reusable reservoir 2 to the supply container 5. Furthermore, second connection port 21 is also configured to fluidly connect the inner volume 2a of the reusable reservoir 2 to a cleansing agent container 16.

Cleansing agent container 16 provides a cleansing agent for cleansing the inner volume 2a of the reusable reservoir 2. For example, a cleansing agent from the cleansing agent container 16 can be conveyed via the second connection port 21 into the inner volume 2a of the reusable reservoir 2 so to flush the inner volume 2a. In order to prevent an unintentional flow of liquid medication into the cleansing agent container and/or to prevent cleansing agent flowing from the cleansing agent container 16 into the supply container 5, a valve 19 is provided. Valve 19 is configured to alternately fluidly connect either the supply container 5 to the inner volume 2a of the reusable reservoir or the cleansing agent container 16 to the inner volume 2a of the reusable reservoir 2.

The discharging device 8 further comprises a heating module 17 that is configured for evaporating deposit in the inner volume 2a of the reusable reservoir 2. The evaporated deposit can than discharged from the reusable reservoir 2a of the reusable reservoir 2. Evaporated deposit can be discharged into the ambient air via an air-vent (not shown) or into the waste container 15.

Furthermore, the filling apparatus 1a comprises a charging device 22 that is configured for charging a rechargeable battery of a reusable reservoir 2 and/or a rechargeable battery of a medical dispensing device 3.

### List of reference numbers

- 1: medical dispensing device system
- 1a: filling apparatus
- 2: reusable reservoir
- 2a: inner volume of reusable reservoir
- 2b: plunger
- 2c: plunger rod
- 3: medical dispensing device
- 4: receiving portion
- 5: supply container
- 6: first connection port
- 7: sterilization device
- 8: discharging device
- 9: radiation chamber
- 10: access opening
- 11: protective barrier
- 12: radiation source
- 13: safety-mechanism
- 14: tamper-evident closure
- 15: waste container
- 16: cleansing agent container
- 17: heating module
- 19: valve
- 20: drive mechanism
- 21: second connection port
- 22: charging device

## Claims

1. A filling apparatus (1a) for filling a reusable reservoir (2) of a medical dispensing device (3), the filling apparatus (1a) comprises:
• a receiving portion (4) that is configured for holding a reusable reservoir (2) of a medical dispensing device (3);
• a supply container (5) that is configured for containing a liquid medication;
• a first connection port (6) that is configured for fluidly connecting an inner volume (2a) of the reservoir (2) of a medical dispensing device (3) with the supply container (5);
• a sterilization device (7) that is configured for sterilizing the inner volume (2a) of the reservoir (2) of a medical dispensing device (3), **characterized in that**
• the filling apparatus (1) comprises a discharging device (8) that is configured to discharge deposit, during use of the filling apparatus (1a), from the reusable reservoir (2) of the medical dispensing device (3);
∘the discharging device (8) is configured for fluidly connecting the inner volume (2a) of the reusable reservoir (2) of a medical dispensing device (3) with the discharging device (8);
• and **in that** the sterilization device (7) comprises a radiation chamber (9) that is configured for housing the reusable reservoir (2) of a medical dispensing device (3); the radiation chamber (9) comprises
∘ an access opening (10) that is configured to be opened or closed; and
∘ a protective barrier (11) that is configured to hinder radiation from leaving the radiation chamber (9);
• and further **in that** the sterilization device (7) comprises a radiation source (12) that is configured to provide radiation within the radiation chamber (9).

2. Filling apparatus (1a) according to claim 1, **characterized in that** the sterilization device (7) comprises a safety-mechanism (13) that switch-off and/or hinder activation of the radiation source (12) in case the access opening (10) of the radiation chamber (9) is open.

3. Filling apparatus (1a) according to claim 1 or 2, **characterized in that** the radiation source (12) comprises or is one or more of a source for UV-C radiation, a source for gamma radiation or a source for X-ray radiation.

4. Filling apparatus (1a) according to any one of claims 1 to 3, **characterized in that** the supply container (5) is a disposable and replaceable supply container comprising a tamper-evident closure (14) that is configured for indicating a first-time opening of the supply container and/or that is configured for hindering refilling of the disposable, replaceable supply container (5).

5. Filling apparatus (1a) according to any one of claims 1 to 4, **characterized in that** the discharging device (8) comprises a waste container (15) that is configured for storing deposit that during use of the filling apparatus (1a) is discharged from the reusable reservoir (2) of the medical dispensing device (3).

6. Filling apparatus (1a) according to claim 5, **characterized in that** the waste container (15) is a removable and disposable container.

7. Filling apparatus (1a) according to any one of claims 1 to 6, **characterized in that** the filling apparatus (1a) comprises a drive mechanism (20) that is configured for actuating a discharging mechanism of the reusable reservoir (2).

8. Filling apparatus (1a) according to any one of claims 1 to 7, **characterized in that** the filling apparatus (1a) comprises a cleansing agent container (16) that is configured for containing a cleansing agent for cleansing an inner volume (2a) of the reusable reservoir (2) of a medical dispensing device (3), wherein, during use of the filling apparatus (1a), the cleansing agent container (16) is fluidly connected to the inner volume (2a) of the reusable reservoir (2) of a medical dispensing device (3).

9. Filling apparatus (1a) according to any one of claims 1 to 8, **characterized in that** the discharging device (8) comprises a heating module (17) that is configured for evaporating deposit in the inner volume (2a) of the reusable reservoir (2) of the medical dispensing device (3).

10. Filling apparatus (1a) according to any one of claims 1 to 9, **characterized in that** the filling apparatus (1a) comprises a charging device (22) for charging a rechargeable battery of a reusable reservoir (2) and/or a rechargeable battery of a medical dispensing device (3).

11. Medical dispensing device system (1) comprising a reusable reservoir (2) for a medical dispensing device (3), and a filling apparatus (1a) for filling the reusable reservoir (2) of a medical dispensing device (3) according to any one of claims 1 to 10.

12. Method for filling a reusable reservoir (2) of a medical dispensing device (3), the method being constituted to be executed by a filling apparatus (1a) according to any one of claims 1 to 10, the method comprises the steps of:
∘ discharging deposit from a reusable reservoir (2) by the discharging device (8) of the filling apparatus (1a);
∘ sterilizing the inner volume (2a) of the reusable reservoir (2) by providing radiation within the radiation chamber (9) by a radiation source (12) of a sterilization device (7) of the filling apparatus (1a); and
∘ filling the inner volume (2a) of the reusable reservoir (2) by providing liquid medication from the supply container (5) of the filling apparatus (1a) via the first connection port (6) of the filling apparatus (1a).

13. Method for filling a reusable reservoir (2) of a medical dispensing device (3) according to claim 12, **characterized in that** the method further comprises the step of:
• evaporating residual deposit in the inner volume (2a) of the reusable reservoir (2) by a heating module (17) of the discharging device (8) of the filling apparatus (1a).

14. Method for filling a reusable reservoir (2) of a medical dispensing device (3) according to claim 12 or 13, **characterized in that** the method further comprises the step of:
• cleansing the inner volume (2a) of the reusable reservoir (2) by flushing the inner volume (2a) of the reusable reservoir (2) with a cleansing agent provided by a cleansing agent container (16) of the filling apparatus (1a).

15. Method for filling a reusable reservoir (2) of a medical dispensing device (3) according to any of claims 12 to 14, **characterized in that** the method further comprises the step of:
• charging a rechargeable battery of the reusable reservoir (2) and/or charging a rechargeable battery of a medical dispensing device (3) by a charging device (22) of the filling apparatus (1a).

## Patentansprüche

1. Füllvorrichtung (1a) zum Befüllen eines wiederverwendbaren Vorratsbehälters (2) einer medizinischen Abgabevorrichtung (3), wobei die Füllvorrichtung (1a) Folgendes umfasst:
• einen Aufnahmeabschnitt (4), der zum Halten eines wiederverwendbaren Vorratsbehälters (2) einer medizinischen Abgabevorrichtung (3) konfiguriert ist;
• einen Versorgungsbehälter (5), der zum Fassen eines flüssigen Medikaments konfiguriert ist;
• einen ersten Verbindungsanschluss (6), der zur fluidischen Verbindung eines Innenvolumens (2a) des Vorratsbehälters (2) einer medizinischen Abgabevorrichtung (3) mit dem Versorgungsbehälter (5) konfiguriert ist;
• eine Sterilisationsvorrichtung (7), die zum Sterilisieren des Innenvolumens (2a) des Vorratsbehälters (2) einer medizinischen Abgabevorrichtung (3) konfiguriert ist, **dadurch gekennzeichnet, dass**
• die Füllvorrichtung (1) eine Abführvorrichtung (8) umfasst, die zum Abführen von Ablagerungen aus dem wiederverwendbaren Vorratsbehälter (2) der medizinischen Abgabevorrichtung (3) während der Verwendung der Füllvorrichtung (1a) konfiguriert ist;
∘ die Abführvorrichtung (8) zur fluidischen Verbindung des Innenvolumens (2a) des wiederverwendbaren Vorratsbehälters (2) einer medizinischen Abgabevorrichtung (3) mit der Abführvorrichtung (8) konfiguriert ist;
• und dadurch, dass die Sterilisationsvorrichtung (7) eine Strahlungskammer (9) umfasst, die zum Unterbringen des wiederverwendbaren Vorratsbehälters (2) einer medizinischen Abgabevorrichtung (3) konfiguriert ist; wobei die Strahlungskammer (9) Folgendes umfasst:
∘ eine Zugangsöffnung (10), die konfiguriert ist, um geöffnet oder geschlossen zu werden; und
∘ eine Schutzbarriere (11), die konfiguriert ist, um zu verhindern, dass Strahlung die Strahlungskammer (9) verlässt;
• und ferner dadurch, dass die Sterilisationsvorrichtung (7) eine Strahlungsquelle (12) umfasst, die zum Bereitstellen von Strahlung innerhalb der Strahlungskammer (9) konfiguriert ist.

2. Füllvorrichtung (1a) nach Anspruch 1, **dadurch gekennzeichnet, dass** die Sterilisationsvorrichtung (7) einen Sicherheitsmechanismus (13) umfasst, der die Aktivierung der Strahlungsquelle (12) ausschaltet und/oder verhindert, falls die Zugangsöffnung (10) der Strahlungskammer (9) offen ist.

3. Füllvorrichtung (1a) nach Anspruch 1 oder 2, **dadurch gekennzeichnet, dass** die Strahlungsquelle (12) eine Quelle für UV-C-Strahlung, eine Quelle für Gammastrahlung oder eine Quelle für Röntgenstrahlung umfasst oder eine oder mehrere davon ist.

4. Füllvorrichtung (1a) nach einem der Ansprüche 1 bis 3, **dadurch gekennzeichnet, dass** der Versorgungsbehälter (5) ein Einweg- und austauschbarer Versorgungsbehälter ist, der einen Originalitätsverschluss (14) umfasst, der zum Anzeigen eines erstmaligen Öffnens des Versorgungsbehälters konfiguriert ist und/oder der zum Verhindern des Wiederbefüllens des Einweg- und austauschbaren Versorgungsbehälters (5) konfiguriert ist.

5. Füllvorrichtung (1a) nach einem der Ansprüche 1 bis 4, **dadurch gekennzeichnet, dass** die Abführvorrichtung (8) einen Abfallbehälter (15) umfasst, der zum Lagern von Ablagerungen konfiguriert ist, die während der Verwendung der Füllvorrichtung (1a) aus dem wiederverwendbaren Vorratsbehälter (2) der medizinischen Abgabevorrichtung (3) abgeführt werden.

6. Füllvorrichtung (1a) nach Anspruch 5, **dadurch gekennzeichnet, dass** der Abfallbehälter (15) ein abnehmbarer Einwegbehälter ist.

7. Füllvorrichtung (1a) nach einem der Ansprüche 1 bis 6, **dadurch gekennzeichnet, dass** die Füllvorrichtung (1a) einen Antriebsmechanismus (20) umfasst, der zum Betätigen eines Abführmechanismus des wiederverwendbaren Vorratsbehälters (2) konfiguriert ist.

8. Füllvorrichtung (1a) nach einem der Ansprüche 1 bis 7, **dadurch gekennzeichnet, dass** die Füllvorrichtung (1a) einen Reinigungsmittelbehälter (16) umfasst, der zum Fassen eines Reinigungsmittels zum Reinigen eines Innenvolumens (2a) des wiederverwendbaren Vorratsbehälters (2) einer medizinischen Abgabevorrichtung (3) konfiguriert ist, wobei während der Verwendung der Füllvorrichtung (1a) der Reinigungsmittelbehälter (16) fluidisch mit dem Innenvolumen (2a) des wiederverwendbaren Vorratsbehälters (2) einer medizinischen Abgabevorrichtung (3) verbunden ist.

9. Füllvorrichtung (1a) nach einem der Ansprüche 1 bis 8, **dadurch gekennzeichnet, dass** die Abführvorrichtung (8) ein Heizmodul (17) umfasst, das zum Verdampfen von Ablagerungen in dem Innenvolumen (2a) des wiederverwendbaren Vorratsbehälters (2) der medizinischen Abgabevorrichtung (3) konfiguriert ist.

10. Füllvorrichtung (1a) nach einem der Ansprüche 1 bis 9, **dadurch gekennzeichnet, dass** die Füllvorrichtung (1a) eine Ladevorrichtung (22) zum Laden einer wiederaufladbaren Batterie eines wiederverwendbaren Vorratsbehälters (2) und/oder einer wiederaufladbaren Batterie einer medizinischen Abgabevorrichtung (3) umfasst.

11. Medizinisches Abgabevorrichtungssystem (1), umfassend einen wiederverwendbaren Vorratsbehälter (2) für eine medizinische Abgabevorrichtung (3) und eine Füllvorrichtung (1a) zum Befüllen des wiederverwendbaren Vorratsbehälters (2) einer medizinischen Abgabevorrichtung (3) nach einem der Ansprüche 1 bis 10.

12. Verfahren zum Befüllen eines wiederverwendbaren Vorratsbehälters (2) einer medizinischen Abgabevorrichtung (3), wobei das Verfahren ausgebildet ist, um durch eine Füllvorrichtung (1a) nach einem der Ansprüche 1 bis 10 ausgeführt zu werden, wobei das Verfahren die folgenden Schritte umfasst:
∘ Abführen von Ablagerungen aus einem wiederverwendbaren Vorratsbehälter (2) durch die Abführvorrichtung (8) der Füllvorrichtung (1a);
∘ Sterilisieren des Innenvolumens (2a) des wiederverwendbaren Vorratsbehälters (2) durch Bereitstellen von Strahlung innerhalb der Strahlungskammer (9) durch eine Strahlungsquelle (12) einer Sterilisationsvorrichtung (7) der Füllvorrichtung (1a); und
∘ Befüllen des Innenvolumens (2a) des wiederverwendbaren Vorratsbehälters (2) durch Bereitstellen von flüssigem Medikament aus dem Versorgungsbehälter (5) der Füllvorrichtung (1a) über den ersten Verbindungsanschluss (6) der Füllvorrichtung (1a).

13. Verfahren zum Befüllen eines wiederverwendbaren Vorratsbehälters (2) einer medizinischen Abgabevorrichtung (3) nach Anspruch 12, **dadurch gekennzeichnet, dass** das Verfahren ferner den folgenden Schritt umfasst:
• Verdampfen von Restablagerungen in dem Innenvolumen (2a) des wiederverwendbaren Vorratsbehälters (2) durch ein Heizmodul (17) der Abführvorrichtung (8) der Füllvorrichtung (1a).

14. Verfahren zum Befüllen eines wiederverwendbaren Vorratsbehälters (2) einer medizinischen Abgabevorrichtung (3) nach Anspruch 12 oder 13, **dadurch gekennzeichnet, dass** das Verfahren ferner den folgenden Schritt umfasst:
• Reinigen des Innenvolumens (2a) des wiederverwendbaren Vorratsbehälters (2) durch Spülen des Innenvolumens (2a) des wiederverwendbaren Vorratsbehälters (2) mit einem Reinigungsmittel, das von einem Reinigungsmittelbehälter (16) der Füllvorrichtung (1a) bereitgestellt wird.

15. Verfahren zum Befüllen eines wiederverwendbaren Vorratsbehälters (2) einer medizinischen Abgabevorrichtung (3) nach einem der Ansprüche 12 bis 14, **dadurch gekennzeichnet, dass** das Verfahren ferner den folgenden Schritt umfasst:
• Laden einer wiederaufladbaren Batterie des wiederverwendbaren Vorratsbehälters (2) und/oder Laden einer wiederaufladbaren Batterie einer medizinischen Abgabevorrichtung (3) durch eine Ladevorrichtung (22) der Füllvorrichtung (1a).

## Revendications

1. Appareil de remplissage (1a) pour le remplissage d'un réservoir réutilisable (2) d'un dispositif de distribution médical (3), l'appareil de remplissage (1a) comprend :
• une partie de réception (4) qui est conçue pour retenir un réservoir réutilisable (2) d'un dispositif de distribution médical (3) ;
• un récipient d'alimentation (5) qui est conçu pour contenir un médicament liquide ;
• un premier orifice de liaison (6) qui est conçu pour relier de manière fluidique un volume interne (2a) du réservoir (2) d'un dispositif de distribution médical (3) au récipient d'alimentation (5) ;
• un dispositif de stérilisation (7) qui est conçu pour stériliser le volume interne (2a) du réservoir (2) d'un dispositif de distribution médical (3), **caractérisé en ce que**
• l'appareil de remplissage (1) comprend un dispositif d'évacuation (8) qui est conçu pour évacuer le dépôt, pendant l'utilisation de l'appareil de remplissage (1a), du réservoir réutilisable (2) du dispositif de distribution médical (3) ;
∘ le dispositif d'évacuation (8) est conçu pour relier de manière fluidique le volume interne (2a) du réservoir réutilisable (2) d'un dispositif de distribution médical (3) au dispositif d'évacuation (8) ;
• et **en ce que** le dispositif de stérilisation (7) comprend une chambre de rayonnement (9) qui est conçue pour accueillir le réservoir réutilisable (2) d'un dispositif de distribution médical (3) ; la chambre de rayonnement (9) comprend
∘ une ouverture d'accès (10) qui est conçue pour être ouverte ou fermée ; et
∘ une barrière de protection (11) qui est conçue pour empêcher le rayonnement de quitter la chambre de rayonnement (9) ;
• et en outre **en ce que** le dispositif de stérilisation (7) comprend une source de rayonnement (12) qui est conçue pour fournir un rayonnement au sein de la chambre de rayonnement (9).

2. Appareil de remplissage (1a) selon la revendication 1, **caractérisé en ce que** le dispositif de stérilisation (7) comprend un mécanisme de sécurité (13) qui éteint et/ou empêche l'activation de la source de rayonnement (12) dans le cas où l'ouverture d'accès (10) de la chambre de rayonnement (9) est ouverte.

3. Appareil de remplissage (1a) selon la revendication 1 ou 2, **caractérisé en ce que** la source de rayonnement (12) comprend ou est une ou plusieurs parmi une source de rayonnement UV-C, une source de rayonnement gamma ou une source de rayons X.

4. Appareil de remplissage (1a) selon l'une quelconque des revendications 1 à 3, **caractérisé en ce que** le récipient d'alimentation (5) est un récipient d'alimentation jetable et remplaçable comprenant une fermeture inviolable (14) qui est conçue pour indiquer une toute première ouverture du récipient d'alimentation et/ou qui est conçue pour empêcher à nouveau le remplissage du récipient d'alimentation jetable et remplaçable (5).

5. Appareil de remplissage (1a) selon l'une quelconque des revendications 1 à 4, **caractérisé en ce que** le dispositif d'évacuation (8) comprend un récipient à déchets (15) qui est conçu pour stocker le dépôt qui pendant l'utilisation de l'appareil de remplissage (1a) est évacué du réservoir réutilisable (2) du dispositif de distribution médical (3).

6. Appareil de remplissage (1a) selon la revendication 5, **caractérisé en ce que** le récipient à déchets (15) est un récipient amovible et jetable.

7. Appareil de remplissage (1a) selon l'une quelconque des revendications 1 à 6, **caractérisé en ce que** l'appareil de remplissage (1a) comprend un mécanisme d'entraînement (20) qui est conçu pour actionner un mécanisme d'évacuation du réservoir réutilisable (2).

8. Appareil de remplissage (1a) selon l'une quelconque des revendications 1 à 7, **caractérisé en ce que** l'appareil de remplissage (1a) comprend un récipient d'agent nettoyant (16) qui est conçu pour contenir un agent nettoyant pour nettoyer un volume interne (2a) du réservoir réutilisable (2) d'un dispositif de distribution médical (3), dans lequel, pendant l'utilisation de l'appareil de remplissage (1a), le récipient d'agent nettoyant (16) est relié de manière fluidique au volume interne (2a) du réservoir réutilisable (2) d'un dispositif de distribution médical (3).

9. Appareil de remplissage (1a) selon l'une quelconque des revendications 1 à 8, **caractérisé en ce que** le dispositif d'évacuation (8) comprend un module de chauffage (17) qui est conçu pour évaporer le dépôt dans le volume interne (2a) du réservoir réutilisable (2) du dispositif de distribution médical (3).

10. Appareil de remplissage (1a) selon l'une quelconque des revendications 1 à 9, **caractérisé en ce que** l'appareil de remplissage (1a) comprend un dispositif de charge (22) pour charger une batterie rechargeable d'un réservoir réutilisable (2) et/ou une batterie rechargeable d'un dispositif de distribution médical (3).

11. Système de dispositif de distribution médical (1) comprenant un réservoir réutilisable (2) pour un dispositif de distribution médical (3), et un appareil de remplissage (1a) pour remplir le réservoir réutilisable (2) d'un dispositif de distribution médical (3) selon l'une quelconque des revendications 1 à 10.

12. Procédé de remplissage d'un réservoir réutilisable (2) d'un dispositif de distribution médical (3), le procédé étant constitué pour être exécuté par un appareil de remplissage (1a) selon l'une quelconque des revendications 1 à 10, le procédé comprend les étapes de :
∘ évacuation du dépôt d'un réservoir réutilisable (2) par le dispositif d'évacuation (8) de l'appareil de remplissage (1a) ;
∘ stérilisation du volume interne (2a) du réservoir réutilisable (2) en fournissant un rayonnement au sein de la chambre de rayonnement (9) par une source de rayonnement (12) d'un dispositif de stérilisation (7) de l'appareil de remplissage (1a) ; et
∘ remplissage du volume interne (2a) du réservoir réutilisable (2) en fournissant un médicament liquide depuis le récipient d'alimentation (5) de l'appareil de remplissage (1a) par l'intermédiaire du premier orifice de liaison (6) de l'appareil de remplissage (1a).

13. Procédé de remplissage d'un réservoir réutilisable (2) d'un dispositif de distribution médical (3) selon la revendication 12, **caractérisé en ce que** le procédé comprend en outre l'étape de :
• évaporation du dépôt résiduel dans le volume interne (2a) du réservoir réutilisable (2) par un module de chauffage (17) du dispositif d'évacuation (8) de l'appareil de remplissage (1a).

14. Procédé de remplissage d'un réservoir réutilisable (2) d'un dispositif de distribution médical (3) selon la revendication 12 ou 13, **caractérisé en ce que** le procédé comprend en outre l'étape de :
• nettoyage du volume interne (2a) du réservoir réutilisable (2) en rinçant le volume interne (2a) du réservoir réutilisable (2) avec un agent nettoyant fourni par un récipient d'agent nettoyant (16) de l'appareil de remplissage (1a).

15. Procédé de remplissage d'un réservoir réutilisable (2) d'un dispositif de distribution médical (3) selon l'une quelconque des revendications 12 à 14, **caractérisé en ce que** le procédé comprend en outre l'étape de :
• chargement d'une batterie rechargeable du réservoir réutilisable (2) et/ou chargement d'une batterie rechargeable d'un dispositif de distribution médical (3) par un dispositif de charge (22) de l'appareil de remplissage (1a).
